(19)

Europäisches
Patentamt
European
Patent Office
Office européen
des brevets

(11) **EP 4 603 007 A1**

(12) **EUROPEAN PATENT APPLICATION**

(43) Date of publication:
**20.08.2025 Bulletin 2025/34**

(21) Application number: **25150189.6**

(22) Date of filing: **03.01.2025**

(51) International Patent Classification (IPC):
**A61B 5/024** (2006.01)   **A61B 5/329** (2021.01)
**A61B 5/347** (2021.01)   **A61B 5/374** (2021.01)
**A61B 5/00** (2006.01)

(52) Cooperative Patent Classification (CPC):
**A61B 5/4884; A61B 5/02405; A61B 5/329;
A61B 5/347; A61B 5/374; A61B 5/7267**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **15.02.2024 JP 2024021289**

(71) Applicant: **TOYOTA JIDOSHA KABUSHIKI
KAISHA**
**Aichi-ken, 471-8571 (JP)**

(72) Inventors:
• **HAYASHI, Keiji**
**Toyota-shi, 471-8571 (JP)**
• **YAMADA, Hitoshi**
**Toyota-shi, 471-8571 (JP)**

(74) Representative: **D Young & Co LLP**
**3 Noble Street**
**London EC2V 7BQ (GB)**

(54) **PHYSIOLOGICAL STATE ESTIMATION SYSTEM, PHYSIOLOGICAL STATE ESTIMATION
METHOD, AND PHYSIOLOGICAL STATE ESTIMATION PROGRAM**

(57)   A physiological state estimation system (1) includes: a feature value calculation unit (201) configured to calculate a feature value from a time-series variation in amplitude and a time-series variation in frequency in each of the frequency bands of a time-series signal representing the physiological state of the living body; a shift vector calculation unit (202) configured to calculate a shift vector indicating a change in the physiological state of the living body in a multidimensional vector space composed of the calculated feature values; a physiological state estimation unit (203) configured to estimate the physiological state of the living body based on a combination ratio of the combined shift vectors before and after an arbitrary physiological state change. The feature value calculation unit (201), the shift vector calculation unit (202), and the physiological state estimation unit (203) can be implemented by using a model trained through machine learning.

Fig. 3

**Description**

BACKGROUND

The present disclosure relates to a physiological state estimation system, a physiological state estimation method, and a physiological state estimation program for estimating the physiological state of a living body.

Various technologies for estimating a physiological state of a living body have been proposed. As an example of such technologies, a physiological state estimation apparatus disclosed in Patent Literature 1 calculates a time-series frequency waveform from a time-series waveform of a living-body signal, and performs a frequency analysis on time-series changes in the inclination of frequencies obtained in each predetermined time window for the time-series frequency waveform. Then, the physiological state estimation apparatus calculates, for each analyzed waveform obtained by the frequency analysis, a determination reference point for the analyzed waveform by using at least one of a region score based on the inclination of a respective regression line of the analyzed waveform and a shape score based on the number of break points of the entire regression line, and estimates the state of the living body based on the time-series changes in the determination reference point of each analyzed waveform.

Patent Literature 1: Japanese Unexamined Patent Application Publication No. 2012-239480

Patent Literature 2: Japanese Patent No. 7327363

Non-patent Literature 1: H. S. Teoh, "Formula for Vector Rotation in Arbitrary Planes in Rn", [online], April 17, 2005, [retrieved on December 14, 2023], Internet <URL: https://www.qfbox.info/4d/genrot.pdf>

SUMMARY

However, the physiological state estimation apparatus disclosed in Patent Literature 1 uses the determination reference point of each analyzed waveform obtained by performing a frequency analysis on time-series changes in the inclination of the time-series frequency waveform based on the living body signal is used as a feature value, therefore, the feature value that can indicate the physiological state of the living body is limited to this determination reference point of each analyzed waveform. Consequently, there has been a problem that it is not possible to estimate changes in various physiological states of a living body.

The present disclosure has been made to solve such a problem, and an object thereof is to provide a physiological state estimation system, a physiological state estimation method, and a physiological state estimation program capable of estimating changes in various physiological states of a living body.

A physiological state estimation system configured to estimate a physiological state of a living body according to an embodiment includes:

a feature value calculation unit configured to divide a time-series signal representing the physiological state of the living body into a plurality of frequency bands and calculate a feature value from a time-series variation in amplitude and a time-series variation in frequency in each of the frequency bands of the time-series signal;
a shift vector calculation unit configured to calculate a shift vector representing the physiological state of the living body in a multidimensional vector space composed of the calculated feature values and indicating a change in the physiological state of the living body in the multidimensional vector space;
a storage unit configured to store shift vectors before and after a plurality of environmental stimuli; and
a physiological state estimation unit configured to combine shift vectors before and after an arbitrary physiological state change by using shift vectors before and after a desired environmental stimulus stored in the storage unit, and estimate the physiological state of the living body based on a combination ratio of the combined shift vectors.

The physiological state estimation unit holds shift vectors in the multidimensional vector space for the plurality of environmental stimuli on a database, expresses shift vectors in the multidimensional vector space in a specific time series by a linear combination of shift vectors for a plurality of environmental stimuli on the database, and estimates a combination coefficient of the linear combination as a ratio of the physiological state.

Specifically, the feature value calculation unit calculates a feature value representing a physiological state of a certain person from his/her biological state time-series data in the morning and that in the evening. Next, the shift vector calculation unit calculates shift vectors in the multidimensional vector space. When the shift vectors can be combined, for example, at a ratio of A:B:C by using three vectors, i.e., a vector of a mental fatigue, a vector of a physical fatigue, and a vector of a rest on the database, the physiological state estimation unit estimates a change in the physiological state of the person from the morning to the evening as a mental fatigue A, a physical fatigue B, and a rest C.

The feature value calculation unit performs a Hilbert transform on waveform information of biological information for each predetermined frequency band as a feature value, and calculates instantaneous amplitudes and instantaneous frequencies of the Hilbert-transformed waveform information. Next, the feature value calculation unit calculates a distribution of instantaneous amplitudes and a distribution of instantaneous frequencies, respectively, in a section which can be regarded as a physiologically steady state, i.e., in a section in which the distributions become unimodal. In the calculated distributions of instantaneous amplitudes and instantaneous frequencies, amplitudes and frequencies are respectively distributed in a Gaussian manner. Then, the feature value calculation unit approximates each of the calculated distributions of instantaneous amplitudes and instantaneous frequencies by a normal distribution, and calculates a mean value and a standard deviation for each of the distributions of instantaneous amplitudes and instantaneous frequencies. In this way, the feature value calculation unit calculates a mean value and a standard deviation of the distribution of instantaneous amplitudes, and a mean value and a standard deviation of the distribution of instantaneous frequencies as feature values representing the physiological state (see Patent Literature 2).

As the frequency band of an autonomic nervous system, HF (High Frequency) (0.15 to 0.4 Hz), which represents the frequency of respiratory fluctuations in heart rate variability, and LF (Low Frequency) (0.04 to 0.15 Hz), which represents the frequency of blood pressure fluctuations, are known. We have confirmed frequency bands VLF (Very Low Frequency) 1 (15 to 40 mHz) and VLF 2 (4 to 15 mHz), which represent autonomic nervous fluctuations at frequencies lower than the aforementioned frequencies, and confirmed that there are frequency bands having the same ratio (i.e., 1/10 times) in even lower frequency bands.

When it is attempted to calculate feature values from autonomic nervous fluctuations at frequencies lower than the VLF 2 band, time-series signals of physiological states of three wavelengths or longer are required to calculate the standard deviation of the distribution of instantaneous amplitudes and the standard deviation of the distribution of instantaneous frequencies in the band of the autonomic nervous fluctuations. For example, in the ULF (Ultra Low Frequency) 1 (1.5 to 4 mHz) band, time-series data of 2,000 seconds (= 3/1.5 mHz) is required. It is difficult to maintain the physiologically steady state for a duration of 30 minutes or longer because the subject could be exposed to various environmental stimuli. Therefore, when feature values are calculated in the four frequency bands HF, LF, VLF 1 and VLF 2, 16 feature values (= 4 bands × 2 (i.e., instantaneous amplitude and instantaneous frequency) × 2 (i.e., mean and standard deviation)) are obtained, so that a 16-dimensional vector space is formed. These feature values are independent of each other.

However, when physiological state time-series data is obtained in a state that can be regarded as a physiologically steady state for a relatively long time such as during sleep, a multidimensional vector space may be formed by calculating feature values in, in addition to the aforementioned frequency bands, frequency bands lower than VLF 2, i.e., frequency bands such as ULF 1 (1.5 to 4 mHz), ULF 2 (0.4 to 1.5 mHz), UULF (Ultra ULF) 1 (0.15 to 0.4 mHz), UULF 2 (0.04 to 0.15 mHz), and the like.

It is possible to determine whether or not the subject is in a physiologically steady state by performing a Hilbert transform on a certain frequency band waveform of a physiological state time-series signal, and determining whether or not its instantaneous amplitudes and instantaneous frequencies are distributed in a Gaussian manner. When a plurality of physiological states are present in the aforementioned time-series signal, the distributions of instantaneous amplitudes and instantaneous frequencies become multimodal distributions.

Note that the physiological state time-series signal may be any biometric data by which information about the physiological state is observed. Specific examples of biometric data include an electrocardiographic waveform, a pulse wave waveform, a cerebral blood flow waveform, an fMRI (functional Magnetic Resonance Imaging) waveform, a far-infrared image, and the like.

When the physiological state time-series data is a pulse wave, pulse interval data can be extracted from the pulse wave, and two time-series data, i.e., a pulse wave series and a pulse wave interval series, can be obtained. Instantaneous phase differences can be defined from these two time-series data. When the distribution of such instantaneous phase differences is calculated, the distribution of instantaneous phase differences becomes a Von Mises distribution or the like in the physiologically steady state, and a mean value and a concentration level of the instantaneous phase differences can be defined. The instantaneous phase differences are important feature values because they contain information about vasoconstriction/vasorelaxation of the autonomic nervous system. Therefore, when a multidimensional vector space is formed by a pulse wave, a 24-dimensional vector space (= 4 bands × 3 (i.e., instantaneous amplitude, instantaneous frequency, and instantaneous phase difference) × (mean and variations)) can be formed (see Patent Literature 2).

Further, a multidimensional vector space may be formed by calculating feature values for frequency bands $\delta$ (0.4 to 4 Hz), $\theta$ (4 to 8 Hz), $\alpha$ (8 to 13 Hz), $\beta$ (13 to 30 Hz), and $\gamma$ (30 Hz and higher) by using physiological state time-series data of a brain wave and a cerebral blood flow.

A method for calculating a database of shift vectors in a multidimensional vector space for a plurality of environmental stimuli will be described. By using data of a population statistically significant for a certain environmental stimulus, feature values before and after the environmental stimulus are calculated. Then, the calculated feature values are mapped onto a multidimensional spatial coordinate system. Note that the feature values are standardized in advance by using data before a plurality of environmental stimuli so that their mean value becomes 0 and their standard deviation becomes 1. This process is performed in order to ensure accuracy when a physiological state is estimated. Next, shift vectors from multidimensional spatial coordinates before an environmental stimulus to multidimensional spatial coordinates after the environmental stimulus is calculated. Then, a mean vector is calculated by averaging the calculated shift vectors for the population, and the calculated mean vector is registered as a shift vector of the environmental stimulus in the database.

The above-described series of processes is carried out, for example, for a mental fatigue, a physical fatigue, a rest, a sleep, a massage, heating, cooling, noises, vibrations, and the like, and feature values before and after the stimuli are calculated for a statistically significant number of subjects. By doing so, shift vectors based on the feature values and information amount distances are recorded in the database.

For example, the above-described standardized feature values representing the physiological state of a certain person is calculated from his/her biological state time-series data in the morning and that in the evening. Next, a shift vector ab based on the above-described standardized feature value in the multidimensional vector space is calculated. When the calculated shift vector can be combined by shift vectors $p_i q_i$ (i = 1, 2, 3, ... ) of a plurality of environmental stimuli on the database, a change between the two physiological states can be expressed by using a combination ratio of the shift vectors $p_i q_i$.

According to the present disclosure, it is possible to provide a physiological state estimation system, a physiological state estimation method, and a physiological state estimation program capable of estimating changes in various physiological states of a living body.

The above and other objects, features and advantages of the present disclosure will become more fully understood from the detailed description given hereinbelow and the accompanying drawings.

BRIEF DESCRIPTION OF DRAWINGS

Fig. 1 shows a configuration of a physiological state estimation apparatus according to an embodiment;
Fig. 2 shows an example of a database generation program for calculating a physiological state shift vector;
Fig. 3 shows an example of a physiological state estimation program;
Fig. 4 is a conceptual diagram showing an example of a shift vector;
Fig. 5 is a flowchart showing an example of a database generation program for calculating a physiological state shift vector;
Fig. 6 is a flowchart showing an example of a physiological state estimation program;
Fig. 7 shows an example of a shift vector resulting from a mental fatigue task;
Fig. 8 shows an example of a shift vector resulting from a physical fatigue task;
Fig. 9 shows an example of a shift vector resulting from a verification experiment;
Fig. 10 shows a relationship among a group of shift vectors resulting from a verification experiment;
Fig. 11 is a conceptual diagram showing information obtained by a Fourier transform and a Hilbert transform;
Fig. 12 is a conceptual diagram of a method for estimating a physiological state according to the present disclosure;
Fig. 13 shows the relationship among the group of shift vectors shown in Fig. 10 when the shift vectors are disposed in an arrangement similar to the vector arrangement shown in in Fig. 12;
Fig. 14 shows a distribution of logarithmic instantaneous amplitudes of an electroencephalogram in each frequency band;
Fig. 15 shows a distribution of instantaneous frequencies of an electroencephalogram in each frequency band; and
Fig. 16 is a diagram for explaining Expression 12 for calculating x and y vectors of Expression 11.

DESCRIPTION OF EMBODIMENTS

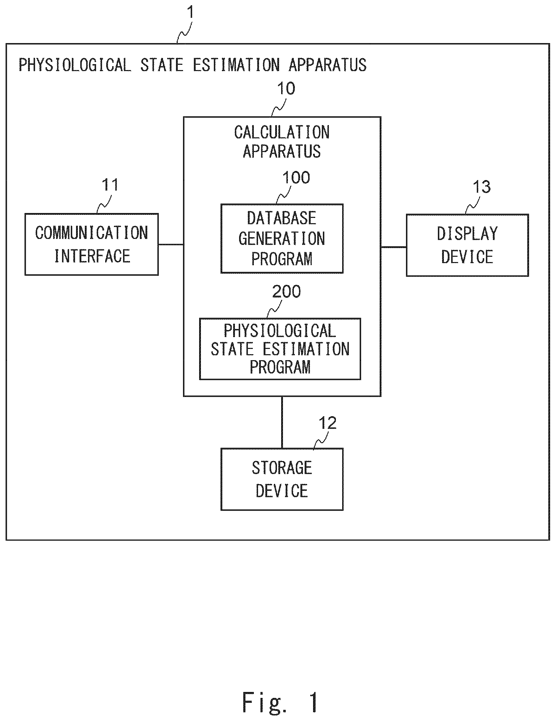

Fig. 1 shows a configuration of a physiological state estimation apparatus 1 according to an embodiment. The physiological state estimation apparatus 1 is an embodiment of a physiological state estimation system. Specific examples of the physiological state estimation apparatus 1 include a PC (Personal Computer), a server, a Raspberry pi, a smartphone, a wearable terminal, a tablet terminal, and an in-vehicle device. Each of these apparatuses corresponds to a computer.

The physiological state estimation apparatus 1 includes a calculation apparatus 10, a communication interface (I/F) 11, a storage device 12, and a display device 13.

The calculation apparatus 10 is an apparatus for performing the overall control of the physiological state estimation apparatus 1. Specific examples of the calculation apparatus 10 include a CPU (Central Processing Unit), an MPU (Micro Processing Unit), and an ECU (Electronic Control Unit). The calculation apparatus 10 corresponds to a computer.

The calculation apparatus 10 executes a database generation program 100 and a physiological state estimation program 200 (which will be described later). Details of the database generation program 100 and the physiological state estimation program 200 will be described later. Note that a semiconductor device such as an FPGA (Field-Programmable Gate Array) or an ASIC (Application Specific Integrated Circuit) may execute the physiological state estimation program. Each of these semiconductor devices also corresponds to a computer.

The communication interface (I/F) 11 is an apparatus for transmitting/receiving data to/from an external apparatus. The communication interface 11 can acquire biological information representing a physiological state of a living body from a sensor for detecting such biological information. Further, the communication interface 11 may also acquire biological information through a network configured by a LAN (Local Area Network) and/or a WAN (Wide Area Network). Further, the biological information may be input from a biological information sensor to the calculation apparatus through a wire and through an analog-to-digital conversion device. Specific examples of sensors for detecting biological information include a pulse wave sensor, a heart rate sensor, a cerebral blood flow sensor such as a near-infrared spectroscopy sensor, and an fMRI capable of observing a blood flow in a brain. Note that in other embodiments, the physiological state estimation apparatus 1 itself may include a sensor for detecting biological information.

The storage device 12 is a storage device storing various information processed by the calculation apparatus 10, such as a database generation program, a physiological state estimation program, a database representing feature values for each physiological state, and physiological measurement data. The display device 13 is an apparatus for displaying various information such as information indicating a physiological state calculated by the calculation apparatus 10. In other embodiments, the display device 13 may be an apparatus separate from the physiological state estimation apparatus 1.

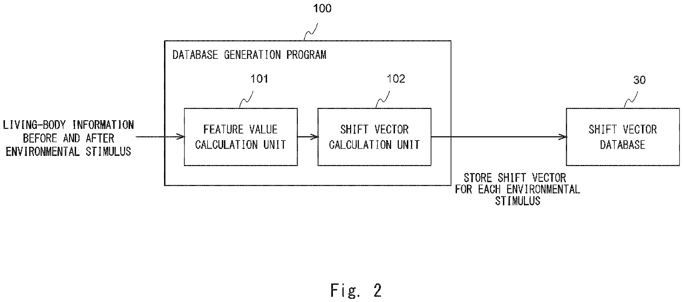

Fig. 2 shows an example of the database generation program 100 for generating a database including, i.e., recording, feature values indicating physiological states resulting from environmental stimuli and shift vectors indicating changes in physiological states. The database generation program 100 includes a feature value calculation unit 101 and a shift vector calculation unit 102.

The feature value calculation unit 101 calculates a feature value indicating a physiological state for each environmental stimulus. Heart rate variability data before and after a certain environmental stimulus, e.g., a mental fatigue task (3-Back task, 20 minutes), are measured for a time that can be regarded as a physiologically steady state, e.g., 10 minutes. Examples of other environmental stimuli may include a physical fatigue task such as climbing up/down a staircase and a treadmill, a nap, a massage, staying in space, and a heating/cooling stimulus. The feature value calculation unit 101 performs a band-pass filter process on a heart rate variability waveform in four frequency bands, e.g., HF (0.15 to 0.4 Hz), LF (0.04 to 0.15 Hz), VLF 1 (15 to 40 mHz), and VLF 2 (4 to 15 mHz). Next, the feature value calculation unit 101 performs a Hilbert transform on each of the waveforms in the respective frequency bands obtained by the band-pass filter process to converts the waveform into a complex number, so that instantaneous amplitudes and instantaneous frequencies can be calculated (see Expressions 4 and 6). When distributions of instantaneous amplitudes and instantaneous frequencies are calculated in a section which can be regarded as a physiologically steady state, i.e., in a section in which the distributions become unimodal, the instantaneous amplitudes and the instantaneous frequencies are distributed in a Gaussian manner. When the feature value calculation unit 101 approximates each of the distributions of instantaneous amplitudes and instantaneous frequencies by a normal distribution function, and calculates a mean value and a standard deviation for each of the distributions, 16 feature values (= 4 (frequency bands) $\times$ 2 (i.e., instantaneous amplitude and instantaneous frequency) $\times$ 2 (i.e., mean and standard deviation)), which are mathematically independent of each other, are obtained. Since physiological states before a plurality of environmental stimuli can be regarded as the same as each other, data before and after the environmental stimuli are standardized so that their mean value becomes 0 and their standard deviation becomes 1. Then, the standardized data are registered in the database. These standardizations are performed in order to ensure the accuracy of the vector combination in the 16-dimensional data space.

The shift vector calculation unit 102 calculates shift vectors in the vector space before and after, for example, a mental fatigue task by using the standardized feature values calculated by the feature value calculation unit 101. A population in which a difference between before and after a task becomes statistically significant, e.g., heartbeat interval measurement data of 30 people, is measured before and after a mental fatigue task, and a shift vector is calculated by performing the above-described standardization. Then, a mean vector of the shift vectors for 30 people is calculated, and defined as a shift vector representing a physiological state change resulting from the mental fatigue task. Further, this shift vector is registered in a shift vector database 30 corresponding to the storage unit. If the coordinate change between before and after the mental fatigue task is not statistically significant, the population is increased until it becomes statistically significant, and then, a shift vector is obtained. Further, heartbeat interval measurement data before and after an environmental stimulus for a specific person may be acquired a plurality of times, and a shift vector may be registered in the database in a similar manner.

In the above-described example, the change in the physiological state resulting from an environmental stimulus is not very large. However, when the change in the physiological state resulting from an environmental stimulus is very large, vectors cannot be accurately combined. In other words, the variance variations of the standardized feature values increase, and the contribution ratio of the feature values of which the variance is large to the vector combination increases, so that the physiological state cannot be accurately estimated. The above-described process is performed in order to ensure the orthogonality between vectors expressing feature values. In such a case, an orthogonal coordinate system is calculated by obtaining an eigenvalue vector matrix by using the above-described standardized feature values before the environmental stimulus recorded in the database, and obtaining the product of matrices of the obtained eigenvalue vector matrix and the above-described standardized feature values before and after the environmental stimulus, and by doing so, the original feature value data is expressed in the orthogonal coordinate system. Next, the transformed shift vector is calculated. Further, the averaged shift vector is calculated for each environmental stimulus, and the averaged shift vector is registered in the shift vector database 30. A PCA or a sweeping method can be adopted as a method for the above-described transformation into the orthogonal coordinate system.

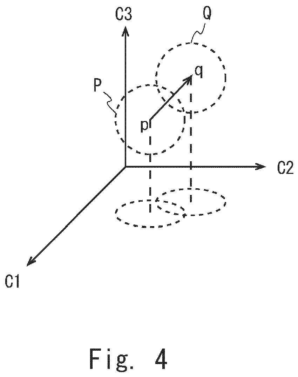

Fig. 4 shows a 16-dimensional physiological state vector space expressed in three dimensions in an imitated manner. The distribution of feature values before and after an environmental stimulus in the 16-dimensional vector space are indicated by P and Q, respectively. Orthogonal coordinate axes C1, C2, C3, ... , and C16 are obtained by performing the above-described standardization or the above-described orthogonalization operation by using the 16-dimensional feature value indicated by P. Next, shift vectors from coordinates before an environmental stimulus to coordinates after the environmental stimulus are calculated in the orthogonal coordinate space, and a shift vector obtained by averaging such vectors for each environmental stimulus is calculated. The obtained shift vector is defined as a shift vector from a P group before the environmental stimulus to a Q group after the environmental stimulus, and registered in the shift vector database 30. A shift vector database 30 can be constructed by repeating the above-described series of operations for each environmental stimulus.

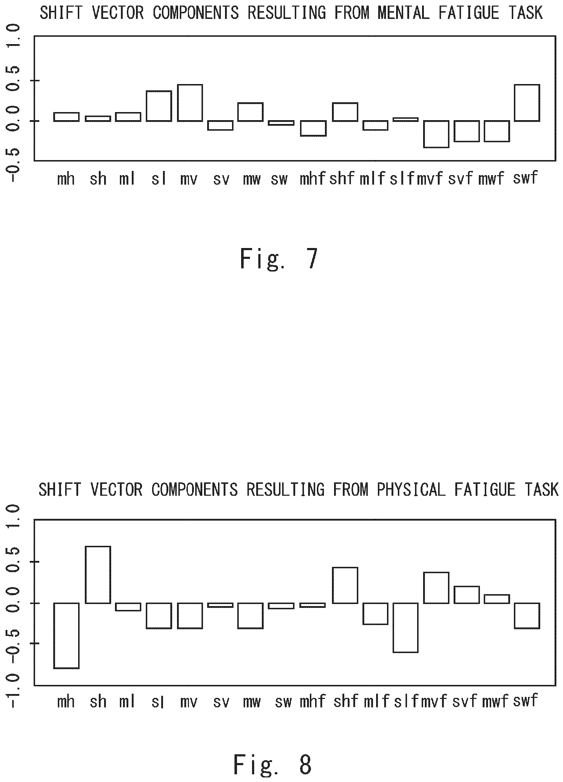

Shift vectors were actually obtained by calculating standardized feature values by using data of 10 minutes heartbeat interval measurement before and after a mental fatigue task (3-Back task, 20 minutes) and a physical fatigue task (climbing up/down a staircase corresponding to five floors × 2), and Figs. 7 and 8 show the results of obtained shift vectors, respectively. Note that mh, sh, ml, sl, mv, sv, mw, sw, mhf, shf, mlf, slf, mvf, svf, mwf, and swf on the horizontal axes indicate the shift vector components of the standardized feature values. The letter m represents a mean; s represents a standard deviation; h represents a HF band; l represents a LF band; v represents a VLF 1 band; and w represents a VLF 2 band. An abbreviation composed of two letters represents a feature value related to the amplitude. An abbreviation composed of three letters with f added thereto represents a feature value related to the frequency.

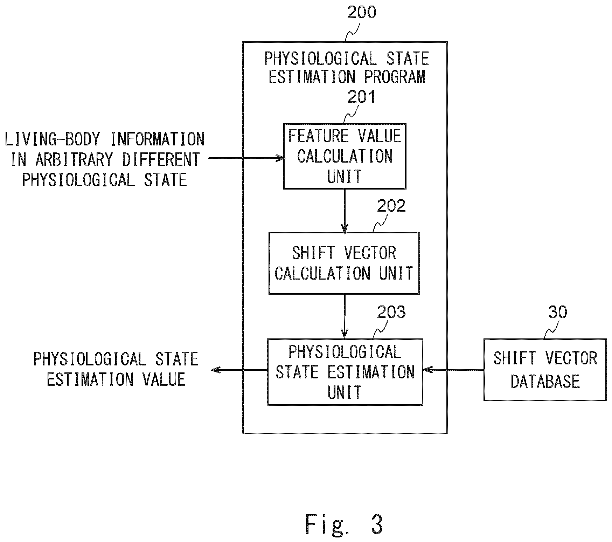

Fig. 3 shows an example of a physiological state estimation program 200. The physiological state estimation program 200 includes a feature value calculation unit 201, a shift vector calculation unit 202, and a physiological state estimation unit 203.

Similarly to the feature value calculation unit 101 described above, the feature value calculation unit 201 is a program for calculating the above-described standardized feature value from biological information which is based on an arbitrary different physiological state. The sensor for acquiring such biological information may be any sensor capable of acquiring a signal containing information about a physiological state, such as a pulse wave sensor, an electrocardiographic sensor, an electroencephalogram sensor, a cerebral blood flow sensor, an fMRI, and an infrared image sensor.

The shift vector calculation unit 202 is a program for calculating a shift vector based on feature values calculated by the feature value calculation unit 201. It is possible to calculate a shift vector from first spatial coordinates to second spatial coordinates by mapping a standardized first feature value and a second feature value onto a 16-dimensional physiological state space.

The physiological state estimation unit 203 is a program for estimating a physiological state by using a shift vector calculated by the shift vector calculation unit 202 and a shift vector stored in the shift vector database 30. Specifically, when the shift vector calculated by the shift vector calculation unit 202 indicates a shift of a shift vector ab from a coordinate to b coordinate in a 16-dimensional vector space, the physiological state estimation unit 203 calculates a vector ab by using shift vectors $D_{ij}$ (i = 1 to 16 (the number of feature values), j = 1 to n (the number of types of environmental stimuli)) of various environmental stimuli stored in the shift vector database 30. The vector ab can be expressed by a linear combination formula shown as Expression 1.

[Expression 1]

$$ab \;=\; \Sigma_i(\,C_i\,) \;=\; \Sigma_i\{\Sigma_j(\,K_j \times D_{ij}\,)\} + \varepsilon \qquad (1)$$

Note that $C_i$ represents a component of the vector ab. $K_j$ represents a coefficient of a linear combination indicating a strength ratio between different environmental stimuli. $\varepsilon$ corresponds to a residual.

The physiological state estimation unit 203 can obtain an optimum coefficient $K_j$ by substituting Expression 2 into Expression 3 and solving n-dimensional linear simultaneous equations based on its partial differentiation. In this way, an arbitrary physiological state can be expressed as a linear combination of coefficients $K_j$ of a plurality of environmental stimuli. Then, $K_j$ can be obtained by solving the minimization problem of Expression 2. For example, $K_j$ can be obtained by solving Expression 3.
[Expression 2]

$$S \;=\; \Sigma_i\{C_i \;-\; \Sigma_j(\,K_j \times D_{ij}\,)\}^2 \qquad (\,2\,)$$

[Expression 3]

$$\delta S \,/\, \delta K_j \;=\; 0 \quad (\,j = 1 \sim n\,) \qquad (\,3\,)$$

Note that Expression 3 represents a partial differentiation.

In the above-described embodiment, a shift vector is obtained by constructing a 16-dimensional vector space for the four frequency bands HF, LF, VLF 1, and VLF 2. However, in other embodiments, the multidimensional vector space may be extended by adopting frequency bands lower than VLF 2, such as ULF 1 (1.5 to 4 mHz), ULF 2 (0.4 to 1.5 mHz), UULF 1 (0.15 to 0.4 mHz), and UULF 2 (0.04 to 0.15 mHz). Further, for physiological measurement data such as a carotid pulse wave, a cerebral blood flow wave, and an fMRI signal, the multidimensional vector space may be extended by adopting frequency bands of brain waves, such as $\delta$ (0.4 to 4 Hz), $\theta$ (4 to 8 Hz), $\alpha$ (8 to 13 Hz), $\beta$ (13 to 30 Hz), and $\gamma$ (30 Hz and higher). Further, frequency bands of brain waves may be divided into a plurality of sections.

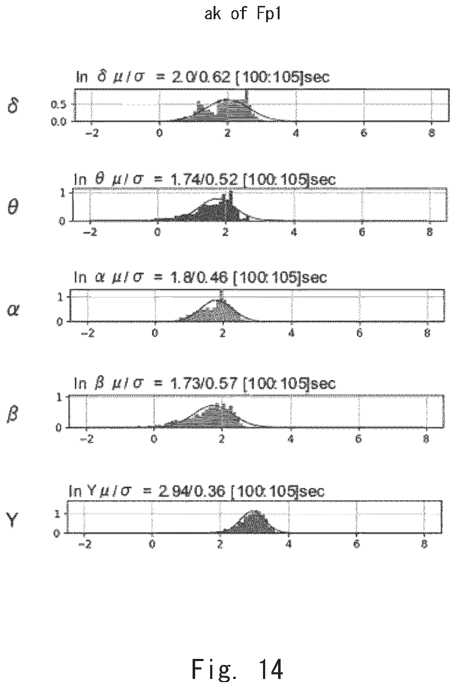

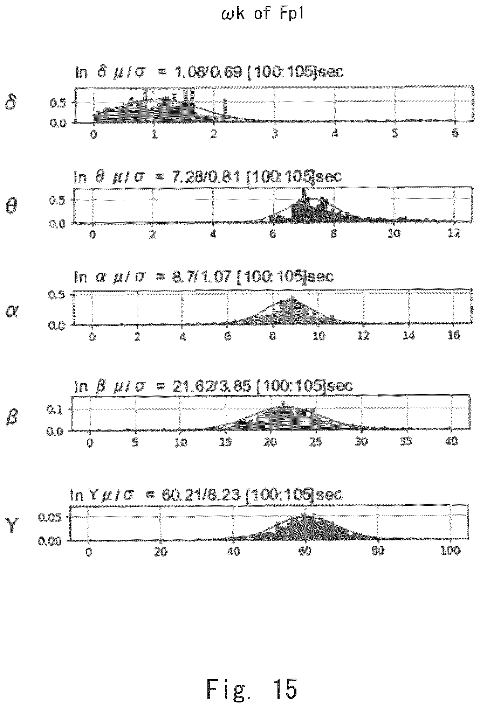

When brain waves are divided into frequency the aforementioned bands $\delta$, $\theta$, $\alpha$, $\beta$ and $\gamma$, and instantaneous amplitudes and instantaneous frequencies are calculated by performing a Hilbert transform on waveforms in each of the bands, they distribute as shown in Figs. 14 and 15. Logarithmic instantaneous amplitudes and instantaneous frequencies in the frequency bands $\delta$, $\theta$, $\alpha$, $\beta$ and $\gamma$ are both distributed in a Gaussian manner. When a mean value and a standard deviation are calculated by approximating these distributions with a Gaussian function, 20 feature values, i.e., 5 (frequency bands) $\times$ 2 (logarithmic instantaneous amplitude and instantaneous frequency) $\times$ 2 (mean and standard deviation) = 20, are obtained, and they make emotion estimation and mental state estimation possible.

Further, by using pulse wave signals, the feature values of phase differences can be defined from two types of related waveforms, i.e., pulse interval waveforms and pulse wave waveforms. Specifically, a vector space equivalent to heartbeat interval data is formed from the pulse interval data. Meanwhile, changes in the amount of pumped-out blood and the state of tension and relaxation of muscles around blood vessels can be observed from the pulse wave waveform data. These changes are observed as phase differences between pulse intervals and pulse wave waveforms (see Patent Literature 2).

When the pulse interval waveform is represented by $\Phi$ (t); the instantaneous amplitude is represented by $A_k$; the instantaneous phase is represented by $\Psi_k$ (t); the instantaneous frequencies is represented by $\Omega_k$ (t); the pulse wave waveform is represented by $\varphi$ (t); the instantaneous amplitude is represented by $a_k$; the instantaneous phase is represented by $\psi_k$ (t); the instantaneous frequency is represented by $\omega_k$ (t); and the instantaneous phase difference is $\theta_k$; k = HF, LF, VLF 1, VLF 2, the pulse interval waveform, the pulse wave waveform, the instantaneous frequency, and the instantaneous phase difference can be expressed by Expressions 4 to 8, respectively.

[Expression 4]
Pulse interval waveform

$$\Phi(t) \;=\; \Sigma exp(\,A_k(t) + i\Psi_k(t)\,) \qquad (\,4\,)$$

[Expression 5]
Pulse waveform

$$\phi(t) \;=\; \Sigma exp(\,a_k(t) + i\psi_k(t)\,) \qquad (\,5\,)$$

[Expression 6]

$$\Omega_k(t) \;=\; d\,\Psi_k(t)\,/\,dt \qquad (\,6\,)$$

[Expression 7]

$$\omega k(t) \;\; = \;\; d\psi k(t) \;\; / \; dt \qquad\qquad (7)$$

[Expression 8]
Phase difference of pulse waveform relative to heartbeat interval waveform

$$\theta k(t) \;\; = \;\; \psi k(t) \; - \; \Psi k(t) \qquad\qquad (8)$$

In a physiologically steady state, the instantaneous amplitudes $A_k$ and $a_k$, and the instantaneous frequencies $\Omega_k$ (t) and $\omega_k(t)$ are distributed in a Gaussian manner, and the instantaneous phase difference $\theta_k$ (t) is distributed in a Von mizese distribution or the like as an angular distribution. Since the feature values corresponding to the mean value and variations can be calculated from these distributions, 24 feature values (= 4 (frequency bands) × 3 (instantaneous amplitude, instantaneous frequency, and instantaneous phase difference) × 2 (mean and variations) can be obtained, so that a 24-dimensional vector space can be constructed. The eight-dimensional feature values increased by Expression 8 represent information about changes in cardiac output and states of tension and relaxation of muscles around blood vessels, and can be used as important physiological feature values. Therefore, by expressing the physiological state in a 24-dimensional vector space including the aforementioned instantaneous phase difference, changes in the physiological state can be recognized in a more detailed manner than in the case where only information about heartbeat intervals are used.

Alternatively, a multidimensional vector space may be formed by performing data processing similar to the above-described processing by using physiological measurement data of brain waves and a cerebral blood flow (such as NIRS), and calculating similar feature values from their amplitudes, frequencies, and phase differences.

The significance of the Hilbert transform of a physiological state signal will be described hereinafter. A ratio LF/HF is usually used to evaluate an autonomic nervous system. When a human being is relaxed, respiratory heart rate variability becomes dominant. Therefore, the tension and relaxation state can be determined based on the magnitude of the power spectral ratio of respiratory heart rate variability HF (0.15 to 0.4 Hz) and blood pressure heart rate variability LF (0.04 to 0.15 Hz) which are obtained by performing a Fourier transform on time-series data of heartbeat intervals. When the heartbeat interval waveforms of the respective frequency bands are expressed as $\varphi_k(t)$, k = HF, LF, VLF 1, VLF 2 ... , the Fourier-transformed signal F is expressed by Expression 9. Meanwhile, the Hilbert-transformed complex signal H is expressed by Expression 10.

[Expression 9]

$$F \;\; = \;\; \Sigma \int \varPhi k(t) exp( -i\omega nt\, )dt \qquad \omega n \in k \qquad (9)$$

[Expression 10]

$$H \;\; = \;\; (1/\pi) \int \varPhi k(t) \; / \; (\, t - \tau\, )d\tau \qquad\qquad (10)$$

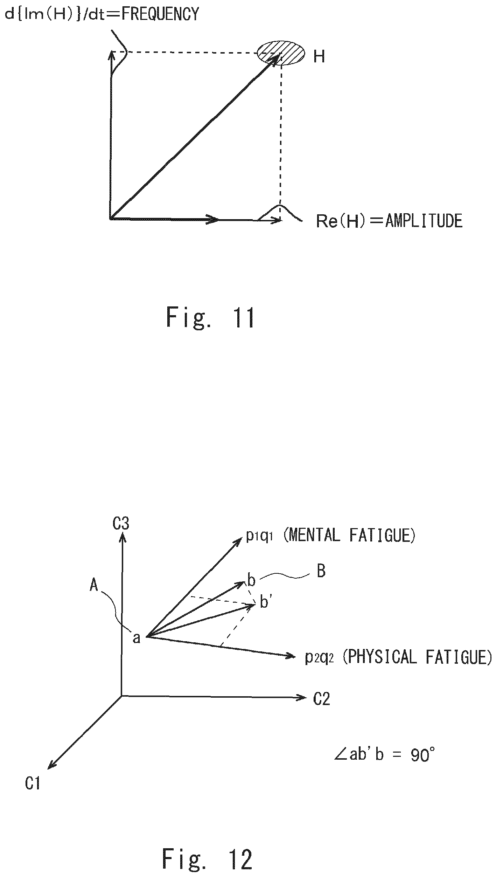

When $\Phi_k$ is Hilbert-transformed, it becomes a time function of the analyzed complex number. The result of the Hilbert transform of $\Phi_k$ in a physiologically steady state is plotted on a graph in which the horizontal axis indicates the real part, and the vertical axis indicates the time differentiation value of the imaginary part as shown in Fig. 11. When the frequency band waveforms of k (k = HF, LF, VLE 1 and VLF 2) of the heartbeat interval is Hilbert-transformed, they become time functions of complex numbers. Their distribution are such distributions that both the real term corresponding to the instantaneous amplitudes and the instantaneous frequencies (the time differentiation of the imaginary term) are distributed in a Gaussian manner in the physiologically steady state, and they are distributed in an elliptical shape on the complex plane.

Since the instantaneous amplitudes and the instantaneous frequencies are distributed in a Gaussian manner in the physiologically steady state, their distributions are approximated by normal distributions and the mean and the variations are obtained therefrom. The amplitudes and frequencies of the distributions of instantaneous amplitudes and instantaneous frequencies, and the mean and the variations are mathematically independent of each other. A wide range of arbitrary physiological states can be expressed by adopting these feature values.

Meanwhile, in the case of the Fourier transform, the time integration of the instantaneous phase term loses its physical meaning, and only the time mean value of the amplitude can be extracted as a physical quantity. However, when the product of the amplitude term and the frequency are time integrated and divided by the time integral value of the amplitude term, the centroid frequency can be obtained. It is also possible to estimate a similar physiological state by using the eight-dimensional feature values (= four frequency bands × 2) obtained as described above. However, it is considered that since the information on the phase in each frequency band is lost, the range of physiological state estimation is limited.

In the above-described embodiment, the physiological state is represented by using 16-dimensional feature values of the mean and standard deviation of the instantaneous amplitudes and the mean and standard deviation of the instantaneous frequencies for the four frequency bands LF, HF, VLF 1, and VLF 2. However, in other embodiments, the physiological state may be expressed by using feature values of dimensions larger than 16 dimensions by using frequency bands lower than VLF 2 or frequency bands higher than HF related to brain activities.

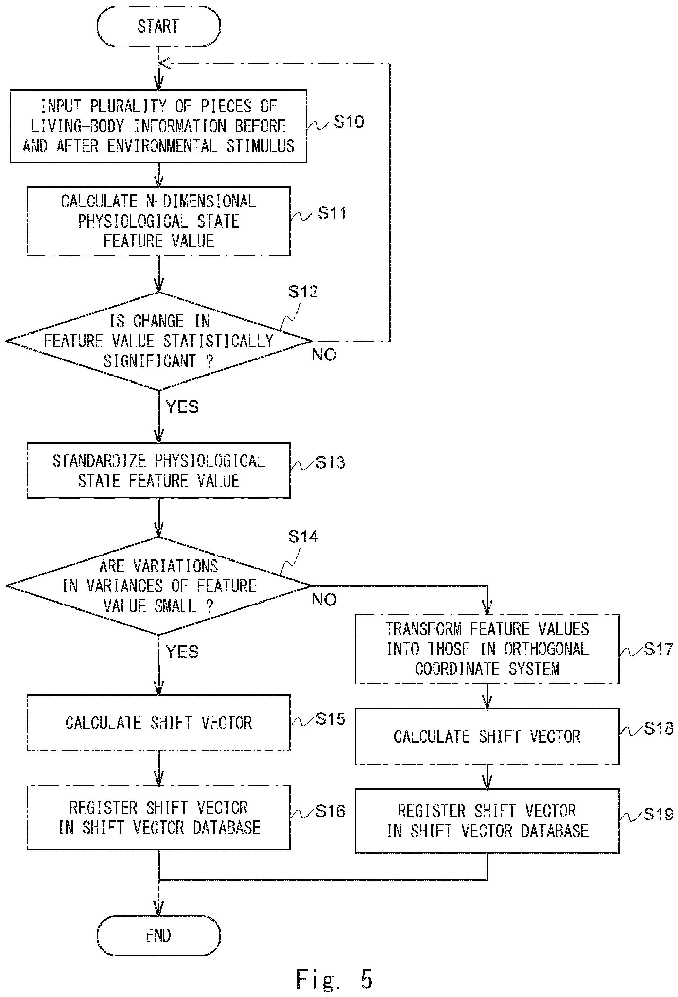

Fig. 5 is a flowchart showing an example of processes that the physiological state estimation apparatus 1 performs based on the database generation program 100.

In a step S10, in order to construct a database, a plurality of physiological state measurement data before and after a specific environmental stimulus are input to the physiological state estimation apparatus 1. In a step S11, after physiological state measurement data is made to pass through a band-pass filter having a desired frequency band, instantaneous amplitudes and instantaneous frequencies are calculated by performing a Hilbert transform and an analysis thereon. Then, a mean value and standard deviation are calculated by approximating their respective distributions by Gaussian functions. By doing so, N physiological state feature values (N = 16 in the case of heartbeat interval data) are calculated. In a step S12, it is determined whether or not the change in the physiological state feature value between before and after the environmental stimulus, calculated in the step S11 is statistically significant. Specifically, e.g., it is determined whether or not the p-value of a t-test or the like for the change in the feature value between before and after the environmental stimulus is equal to or smaller than a predetermined threshold (e.g., 0.05). When it is determined that the change between before and after the environmental stimulus is not significant (No), the process returns to the step S10, and additional biological information is input. When it is determined that the change between before and after the environmental stimulus is significant (Yes), the process proceeds to a step S13.

In a step S13, the data before and after the environmental stimulus are standardized by using the mean value and the standard deviation calculated from a plurality of physiological state feature values before the environmental stimulus already stored in the shift vector database so that the mean value becomes 0 and the standard deviation becomes 1.

In a step S14, the variations of the variance of the standardized N-dimensional feature values before and after the environmental stimulus for each dimension is calculated, and the process branches according to the result of the calculation. When it is determined that the variance variations of each dimension are small (Yes), i.e., when it is determined that the variance variations of each dimension are smaller than or equal to a predetermined threshold, e.g., (variance maximum value - variance minimum value) / variance maximum value ≤ 0.5, the process proceeds to a step S15. On the other hand, when it is determined that the variance variations of each are large (No), i.e., when it is determined that the variance variations of each dimension are larger than the predetermined threshold, e.g., (variance maximum value - variance minimum value) / variance maximum value > 0.5, the process proceeds to a step S17.

In a step S15, a shift vector from before the environmental stimulus to after the environmental stimulus is calculated for each individual data in the N-dimensional spatial coordinate system composed of the above-described standardized feature values before and after the environmental stimulus calculated in the step S13, and an average shift vector averaged for each environmental stimulus is calculated. In a step S16, the averaged shift vector for each environmental stimulus is registered in the database 30, and the series of processes shown in Fig. 5 is finished.

In a step S17, the above-described standardized feature values before and after the environmental stimulus are converted into those in an orthogonal coordinate system. In a step S18, the shift vectors from before the environmental stimulus to after the environmental stimulus are calculated for each individual data, and an average shift vector averaged for each environmental stimulus is calculated. In a step S19, the averaged shift vector for each environmental stimulus is registered in the database 30, and the series of processes shown in Fig. 5 is finished.

When data of a new environmental stimulus is added in the database, or when data is added to the existing environmental stimuli, the processes in the steps S13 to S16 or the steps S13 to S19 have to be performed for the whole data recorded in the database. To do so, it is necessary to register the above-described feature value data which has not been standardized yet and the above-described feature value data which has already been the standardized together with the shift vectors for each environmental stimulus in the shift vector database 30.

Since the shift vectors registered in the step S16 and the shift vectors registered in the step S19 become data spaces completely different from each other, it is necessary to distinguish which physiological state space should be used to estimate the physiological state. When the environmental stimulus used for estimating a physiological state does not include the environmental stimulus registered in the step S19, it can be estimated by the physiological state space registered in the step S16. However, when it includes the environmental stimulus registered in the step S19, it has to be estimated by the physiological state space registered in the step S19.

The correspondence between Figs. 2 and 5 will be described. The feature value calculation unit 101 in Fig. 2 performs the processes in the steps S10 to S13 shown in Fig. 5. The shift vector calculation unit performs the processes in the steps S14 to S19.

Examples of shift vectors obtained for each environmental stimulus are shown in Figs. 7 and 8. Fig. 7 shows a shift vector based on 16-dimensional feature values. These 16-dimensional feature values are calculated with heartbeat interval data that are obtained by carrying out electrocardiographic measurement for 10 minutes before and after a fatigue task (3-Back task, 20 minutes) on each of 37 subjects. This is tentatively defined as a mental fatigue. Fig. 8 shows a shift vector obtained as a result of electrocardiographic measurement for 10 minutes before and after a fatigue task (climbing up/down a staircase corresponding to five floors × 2 times) carried out for each of 46 subjects. This is tentatively defined as a physical fatigue. Note that in each of Figs. 7 and 8, mh, sh, ml, sl, mv, sv, mw, sw, mhf, shf, mlf, slf, mvf, svf, mwf, and swf on the horizontal axes indicate the shift vector components of the standardized feature values. The letter m represents a mean; s represents a standard deviation; h represents a HF band; l represents a LF band; v represents a VLF 1 band; and w represents a VLF 2 band. An abbreviation composed of two letters represents a feature value related to the amplitude. An abbreviation composed of three letters with f added thereto represents a feature value related to the frequency. The vertical axis indicates shift amounts that are standardized so that their standard deviation becomes 1.

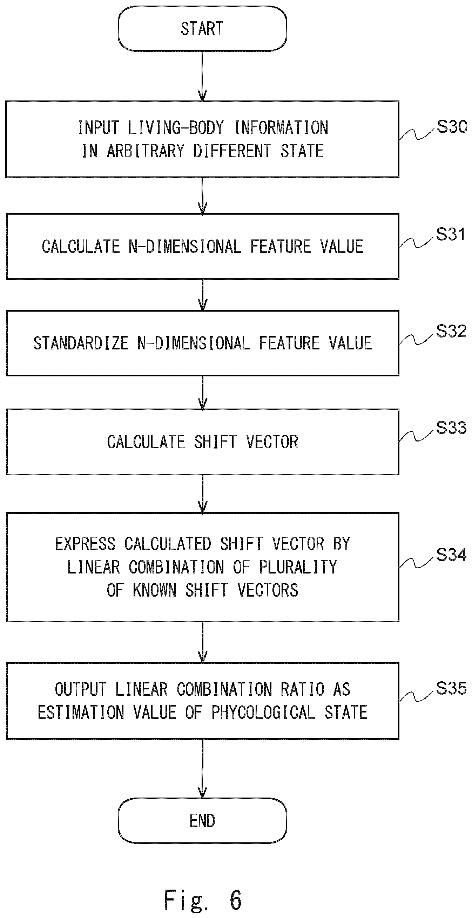

Fig. 6 is a flowchart showing an example of processes that the physiological state estimation apparatus 1 performs based on the physiological state estimation program 200.

In a step S30, physiological state measurement data indicating a first physiological state and a second physiological state are input to the physiological state estimation apparatus 1. In a step S31, the above-described feature values are calculated from the first and second physiological state measurement data. In a step S32, the feature values in the first and second physiological states calculated in the step S31 are standardized by using feature values in physiological states before a plurality of environmental stimulus, recorded in the shift vector database 30. In a step S33, the first and second feature values are mapped into an N-dimensional space, and a shift vector indicating a shift from first spatial coordinates to second spatial coordinates is calculated.

In a step S34, the shift vector calculated in the step S33 is expressed as a linear combination of a plurality of known shift vectors for an environmental stimulus stored in the shift vector database 30, and its combination coefficient is solved as a mathematical optimization problem. In a step S35, a linear combination ratio based on the calculated combination coefficient is output as a physiological state estimation value, and the series of processes shown in Fig. 6 is finished. By adopting the linear combination ratio calculated based on the highly accurate shift vector as a physiological state estimation value as described above, the reliability of the physiological state estimation value can be improved. Regarding the correspondence with the steps shown in Fig. 3, the steps S31 and S32 are performed by the feature value calculation unit 201; the step S33 is performed by the shift vector calculation unit 202; and steps S34 and S35 are performed by the physiological state estimation unit 203.

Specifically, it is assumed that heartbeat interval measurement data of a certain person in the morning and evening are measured, and the shift vector of the physiological state from the morning to the evening is a shift vector ab. Further, a shift vector of a mental fatigue stored in the shift vector database 30 is represented by S and a shift vector of a physical fatigue is represented by R. Then, mathematical optimization is performed by using Expressions 1, 2, and 3, and the coefficient $K_j$ of the linear combination is obtained.

[Expression 11]

$$ab \;=\; K_1 \times S \;+\; K_2 \times R \;+\; \varepsilon \qquad (1\,1)$$

In the expression, $\varepsilon$ represents a residual. Therefore, in the mathematical optimization, $K_1$ and $K_2$ with which the residual $\varepsilon$ is minimized are obtained.

In Expression 11, the fatigue level of the certain person from the morning to the evening is expressed by a mental fatigue $K_1$ and a physical fatigue $K_2$. When the residual $\varepsilon$ can be expressed by a shift vector of another environmental stimulus, the mathematical optimization may be performed by adding a shift vector T and a coefficient K3 in the physiological state corresponding to that environmental stimulus. Then, the physiological state may be estimated based on Expression 12 by adding the physiological state vector T other than the mental fatigue and the physical fatigue to the mental fatigue and the physical fatigue.

[Expression 12]

$$ab \;=\; K_1 \times S \;+\; K_2 \times R \;+\; K_3 \times T \;+\; \varepsilon' \qquad (1\,2)$$

Further, even when the types of environmental stimuli are increased, the physiological state can be estimated in a similar manner.

The state of the above-described operation will be described with reference to a vector diagram shown in Fig. 12. Assume that in the above-described standardized 16-dimensional orthogonal coordinate system, when the state changes from a physiological state A before an environmental stimulus to a physiological state B after an arbitrary environmental stimulus, the position coordinates of these states are represented by coordinates a and b, respectively. When an ab vector, which is a physiological state shift vector, is estimated by using a mental fatigue vector p1q1 and a physical fatigue vector p2q2, and an ab' vector obtained by projecting the ab vector onto a plane including position coordinates p1, q1, p2 and q2 is combined by using the p1q1 vector and the p2q2 vector, its composite ratio becomes the estimated value of the physiological state. In this process, an error occurs between b and b', and this error corresponds to the residual $\varepsilon$.

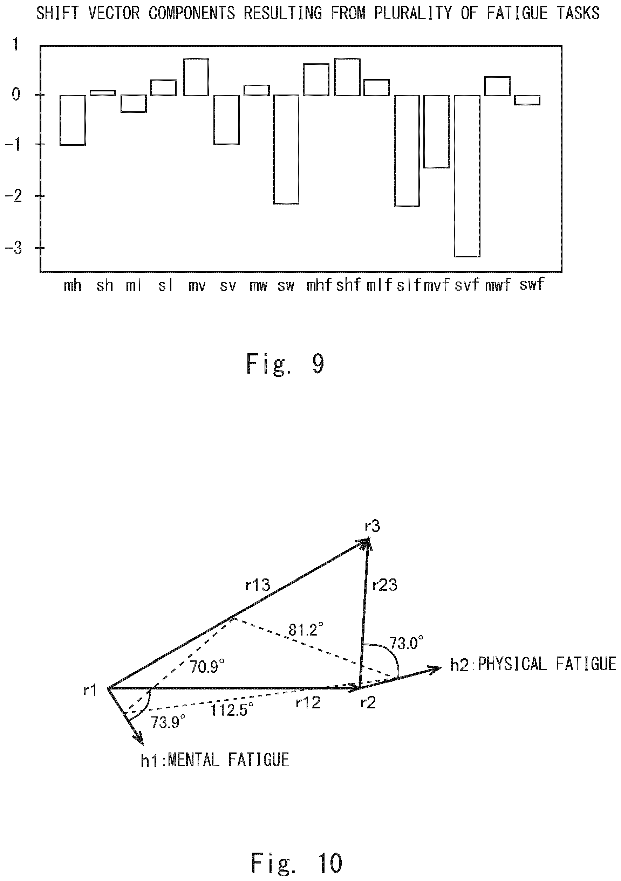

A specific example of the above-described physiological state estimation method will be described hereinafter. For a given subject, a state before an environmental stimulus is represented by r1. Electrocardiography is performed for 10 minutes in a resting state, and then a 3-Back task is performed for 20 minutes. This state is represented by r2. Electrocardiography is performed for 10 minutes in a resting state, and then climbing up/down a staircase corresponding to five floors is performed twice. This state is represented by r3. Then, electrocardiography is performed for 10 minutes in a resting state. Further, a shift vector r13 from r1 to r3 is calculated from the data of r1 to r3 by standardizing the 16-dimensional feature values as the population set of the 16-dimensional feature values before environmental stimulus in the database. Fig. 9 shows the result of the calculation. Note that mh, sh, ml, sl, mv, sv, mw, sw, mhf, shf, mlf, slf, mvf, svf, mwf, and swf on the horizontal axes indicate the shift vector components of the standardized feature values. The letter m represents a mean; s represents a standard deviation; h represents a HF band; l represents a LF band; v represents a VLF 1 band; and w represents a VLF 2 band. An abbreviation composed of two letters represents the amplitude. An abbreviation composed of three letters with f added thereto represents the frequency. The vertical axis indicates shift amounts that are standardized so that their standard deviation becomes 1.

In the above-described method, when the shift vector shown in Fig. 9 is combined by the shift vector of the mental fatigue shown in Fig. 7 and the physical fatigue shown in Fig. 8, $K_1$ becomes 2.4 ($K_1 = 2.4$) (mental fatigue) and $K_2$ becomes 1.1 ($K_2 = 1.1$) (physical fatigue), and the ratio of the mental fatigue to the physical fatigue of the subject can be quantitatively estimated. When the shift vectors of environmental stimuli other than the shift vectors of the physical fatigue and the mental fatigue are known, the ratios of other physiological states can be estimated by performing a similar mathematical optimization.

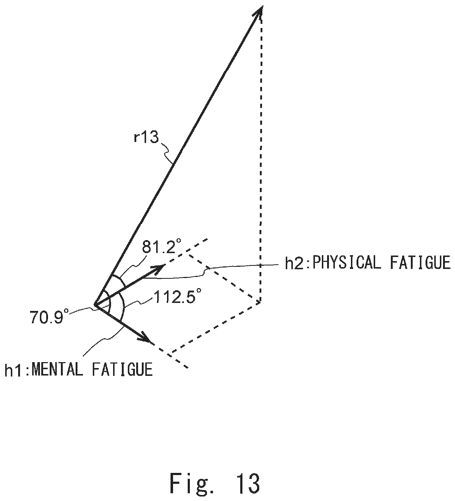

Since the length of a vector and an angle between two vectors in the 16-dimensional space can be found, i.e., determined, their relationships are shown in Figs. 10 and 13. Specifically, the relationship among the position vectors r1 to r3, the mental fatigue vector h1, and the physical fatigue vector h2 is shown in Fig. 10. The angle formed by each of the vectors is also shown in Fig. 10. The angle formed by h1 and h2 is 112.5°. The angle formed by h1 and the vector r12 is 73.9°. The angle formed by h2 and the vector r23 is 73.0°. The angle formed by h1 and the vector r13 is 70.9°. The angle formed by vectors h2 and r13 is 81.2°. Fig. 13 shows the relationship among the group of shift vectors shown in Fig. 10 when the shift vectors are disposed in an arrangement similar to the vector arrangement shown in in Fig. 12.

From these vector diagrams, it can be seen that the error between the r13 vector and the plane formed by the vectors of the mental fatigue and the physical fatigue is large, and the shift of the physiological state resulting from causes other than the shifts of the physiological state resulting from the mental fatigue and the physical fatigue is predicted. By examining the shift vectors of other environmental stimuli, such as a rest, sleep, a massage, heating, cooling, noises, and vibrations, it is expected that components that do not contribute to the vector combination can be combined.

In the above-described embodiment, the physiological state was estimated based on the mental fatigue task and the physical fatigue task. In the physical fatigue task, however, the rest measurement was performed after the exercise, and then rest measurement was performed again, the heart rate rose immediately after the exercise. Therefore, the feature value was calculated by using data for 10 minutes after the heart rate returned to the normal rate after the 5-minute rest state. Therefore, the shift vector of the physical fatigue is a vector obtained by combining the effects of physical fatigue and the fatigue recovery (15 minutes rest), and the shift vector component of the fatigue recovery effect causes an error in the estimated value. In Fig. 10, the angle formed by the shift vectors of the mental fatigue and the physical fatigue is 112.5°. However, there is a possibility that that when the shift vector component of the fatigue recovery effect is eliminated, the shift vector becomes orthogonal (90°), and the relationships become independent of each other. A method for calculating a shift vector of a physical fatigue after the effect of fatigue recovery is eliminated will be described hereinafter.

It is considered that since the shift vector of a mental fatigue is based on feature values calculated from the rest measurement data immediately after the task, factors other than the mental fatigue are hardly mixed therein. Heating and cooling stimuli are considered as candidates for such environmental stimuli. Although such an experiment has not been conducted yet, it is assumed that a shift vector of a heating stimulus has been able to be calculated. Note that it is assumed that the mental fatigue vector and the heating stimulus vector are orthogonal to each other. Then, it is considered that when a 16-dimensional orthogonal coordinate system in which the shift vector of the mental fatigue and the shift vector of the heating stimuli are used as physiological base vectors can be calculated, the shift vector component of the above-described fatigue recovery effect can be eliminated by using, among 14 base vectors excluding the aforementioned two vectors, the base vector closest to the physical fatigue vector, i.e. the base vector of which the angle formed with the shift vector is the smallest, as the physical fatigue vector.

Specifically, the 16-dimensional orthonormal coordinate system used for calculation in the step S15 of Fig. 5 is orthonormalized so that the length becomes 1. Then, the rotational coordinate transformation is performed so that the directions of the mutually orthogonal mental fatigue vector and heating stimulus vector coincide with two axes of the orthonormalized orthonormal coordinate system. For this method, Expression 13 may be used as described in Non-patent Literature 1.

[Expression 13]

$$rot_{P,\phi}(v) = v + \{(v \cdot x)(\cos\phi - 1) - (v \cdot y)\sin\phi\}x + \{(v \cdot y)(\cos\phi - 1) + (v \cdot x)\sin\phi\}y$$

$$(13)$$

This Expression means that an arbitrary 16-dimensional vector v in the 16-dimensional space is rotated by an angle φ on the P-plane that passes through the arbitrarily determined origin. x and y are unit vectors orthogonal to each other on the P-plane, and (v·x) and (v·y) are inner products of vectors. φ is an angle from the normal vector determined by x and y.

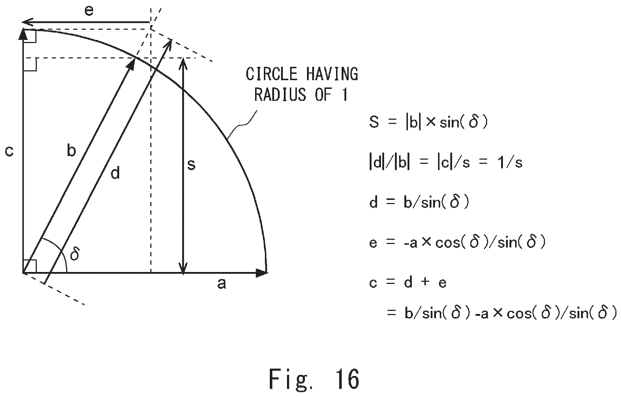

For example, assume that the angle between the mental fatigue base vector b having a length 1 and one of the base vectors of the 16-dimensional orthonormal coordinate system, i.e. a vector a, is δ. In Fig. 16, a vector that forms an angle δ with the vector a is referred to as a vector b, and a vector c having a length 1 orthogonal to the vector a on the plane formed by the vectors a and b is obtained as shown in Expression 14.

[Expression 14]

$$c = b/\sin\delta - a \cdot \cos\delta/\sin\delta \qquad (14)$$

When the mental fatigue vector b is substituted into Expression 11 as x=a, y=c, v=b, φ=-δ in order to perform rotational coordinate transformation of the mental fatigue vector b into the direction of one of the orthonormal vectors, i.e. the vector a, the vector b is subjected to the rotational coordinate transformation and coincides with the vector a.

Next, it is considered that the direction of the heating stimulus vector orthogonal to the aforementioned mental fatigue vector is made to coincide with one of the vectors in the orthonormal system by rotational transformation by using the mental fatigue vector as the axis of the rotation. Specifically, the heating stimulus vector orthogonal to the mental fatigue vector is represented by b; one of the 15 orthonormal vectors of which the direction is different from that of the mental fatigue vector is represented by a; and the angle formed by the one of the 15 orthonormal vectors and the heating stimulus vector is represented by δ. Then, a series of processes similar to the above-described series of processes is performed. Since the vectors a and b are orthogonal to the mental fatigue vector, the rotational coordinate transformation around the mental fatigue vector axis is performed, and a 16-dimensional base vector space including the base vectors of the mental fatigue vector and the heating stimulus vector are obtained. When the base vector, of which the angle with the physical fatigue is smallest among the 14 base vectors other than the mental fatigue vector and the heating stimulus vector, is defined as the physical fatigue vector, it is possible to extract the pure physical fatigue vector component after the physical fatigue task. For each of other environmental stimuli, when an experiment is carried out in the future, the one having the small angle with the shift vector can be defined as the base vector of that environmental stimulus. It has been confirmed that the orthogonality of the orthonormal system is maintained after the rotational coordinate transformation described above.

The above description is given on the assumption that the mental fatigue vector and the heating stimulus vector are physiological base vectors, but they may not be physiological base vectors. It may be possible to define better physiological base vectors by calculating shift vectors of various environmental stimuli in the future.

When the above-described physiological base vectors are obtained, there is no need to solve simultaneous equations composed of Expressions 1 to 3, and shift vectors resulting from arbitrary physiological state changes can be expressed by better base vectors. Therefore, the ratio of each physiological state can be estimated just by obtaining the inner product with the base vector.

In the above-described examples, the program includes instructions (or software codes) that, when loaded into a computer, cause the computer to perform one or more of the functions described in the embodiments. The program may be stored in a non-transitory computer readable medium or a tangible storage medium. By way of example, and not a limitation, non-transitory computer readable media or tangible storage media can include a random-access memory (RAM), a read-only memory (ROM), a flash memory, a solid-state drive (SSD) or other types of memory technologies, a CD-ROM, a digital versatile disc (DVD), a Blu-ray disc or other types of optical disc storage, and magnetic cassettes, magnetic tape, magnetic disk storage or other types of magnetic storage devices. The program may be transmitted on a transitory computer readable medium or a communication medium. By way of example, and not a limitation, transitory computer readable media or communication media can include electrical, optical, acoustical, or other forms of propagated signals.

From the disclosure thus described, it will be obvious that the embodiments of the disclosure may be varied in many ways. Such variations are not to be regarded as a departure from the spirit and scope of the disclosure, and all such modifications as would be obvious to one skilled in the art are intended for inclusion within the scope of the following claims.

<Supplementary notes>

(Supplementary note 1)

A physiological state estimation system comprising:

a feature value calculation unit configured to divide a time-series signal representing the physiological state of the living body into a plurality of frequency bands and calculate a feature value from a time-series variation in amplitude and a time-series variation in frequency in each of the frequency bands of the time-series signal;

a shift vector calculation unit configured to calculate a shift vector representing the physiological state of the living body in a multidimensional vector space composed of the calculated feature values and indicating a change in the physiological state of the living body in the multidimensional vector space;

a storage unit configured to store shift vectors before and after a plurality of environmental stimuli; and

a physiological state estimation unit configured to combine shift vectors before and after an arbitrary physiological state change by using shift vectors before and after a desired environmental stimulus stored in the storage unit, and estimate the physiological state of the living body based on a combination ratio of the combined shift vectors.

(Supplementary note 2)

The physiological state estimation system according to Supplementary note 1, wherein the shift vectors stored in the storage unit are obtained by:

calculating positional coordinates in the multidimensional vector space of feature values based on time-series signals representing physiological states before and after a plurality of types of environmental stimuli for each of a plurality of persons or each of a plurality of times; and

averaging, for each environmental stimulus, a shift vector from before the environmental stimulus to after the environmental stimulus in a state where the change in the physiological state of the living body between before and after the environmental stimulus becomes statistically significant.

(Supplementary note 3)

The physiological state estimation system according to Supplementary note 1, wherein the feature value calculation unit calculates a mean value and a standard deviation by using a plurality of feature values before an environmental stimulus stored in the storage unit, and use the calculated mean value and the standard deviation to standardize data before and after the environmental stimulus so that their mean value becomes 0 and their standard deviation becomes 1.

(Supplementary note 4)

The physiological state estimation system described in Supplementary note 3, wherein

the feature value calculation unit calculates a first feature value by using a time-series signal representing a first physiological state, the first feature value being a standardized feature value before an environmental stimulus,

the feature value calculation unit calculates a second feature value by using a time-series signal representing a second physiological state, the second feature value being a standardized feature value after the environmental stimulus,

the shift vector calculation unit calculates a shift vector representing a change from the first physiological state to the second physiological state in the multidimensional vector space based on the first and second feature values, and

the physiological state estimation unit expresses the shift vector representing the change from the first physiological state to the second physiological state by a linear combination using a shift vector stored in the storage unit, and outputs a ratio of a combination coefficient of the linear combination as an estimated value indicating a change in the physiological state of the living body.

(Supplementary note 5)

The physiological state estimation system described in Supplementary note 4, wherein the feature value calculation unit calculates instantaneous amplitudes and instantaneous frequencies of a signal waveform representing a physiological state in a certain frequency band, and calculates a mean value and a standard deviation obtained by adding up calculated instantaneous amplitudes and instantaneous frequencies in a section that is regarded as a physiologically steady state as the feature values.

(Supplementary note 6)

The physiological state estimation system described in Supplementary note 5, wherein

the frequency band represents the physiological state of the living body, and
the feature value calculation unit calculates the feature value for at least four frequency bands, i.e., HF (0.15 to 0.4 Hz) representing a respiratory cycle of the living body, LF (0.04 to 0.15 Hz) representing a blood pressure fluctuation cycle of the living body, and VLF 1 (15 to 40 mHz) and VLF 2 (4 to 15 mHz) representing fluctuations in an autonomic nervous system of the living body.

(Supplementary note 7)

The physiological state estimation system described in Supplementary note 6, wherein

the frequency band includes ULF 1 (0.15 to 0.4 mHz) and ULF 2 (0.04 to 0.15 mHz), and
the feature value calculation unit calculates the feature value for at least one of the ULF 1 and the ULF 2.

(Supplementary note 8)

The physiological state estimation system described in Supplementary note 6, wherein

the frequency band includes frequency bands $\delta$ (0.4 to 4 Hz), $\theta$ (4 to 8 Hz), $\alpha$ (8 to 13 Hz), $\beta$ (13 to 30 Hz), and $\gamma$ (30 Hz and higher), and
the feature value calculation unit calculates the feature value for at least one of the bands $\delta$, $\theta$, $\alpha$, $\beta$ and $\gamma$.

(Supplementary note 9)

The physiological state estimation system described in Supplementary note 3, wherein when variance variations of the standardized feature value before and after the environmental stimulus stored in the storage unit is larger than a predetermined threshold, the shift vector calculation unit converts a feature value space into an orthogonal space and calculates the shift vector.

(Supplementary note 10)

The physiological state estimation system described in any one of Supplementary notes 1 to 3, wherein when there are two shift vectors orthogonal to each other among shift vectors resulting from an environmental stimulus, the physiological state estimation unit calculates a base vector by rotation-transforming a feature value space so that directions of arbitrary two vectors of a multidimensional eigenvalue vector matrix obtained by converting the feature value space before a plurality of environmental stimulus into an orthogonal space coincide with directions of the two shift vectors orthogonal to each other, and estimates a physiological state with inner product values of shift vectors indicating a change from a first physiological state to a second physiological state and respective base vectors, the inner product values being calculated by using a matrix of the calculated base vectors.

(Supplementary note 11)

The physiological state estimation system described in Supplementary note 4, wherein the feature value calculation unit:

divides each of time-series signals of a pulse interval and a blood flow acquired from the living body into a plurality of frequency bands;
calculates, for each of the frequency bands, a distribution of instantaneous amplitudes and a distribution of instantaneous frequencies of the time-series signals of the pulse interval and the blood flow, and calculates a distribution of instantaneous phase differences between the time-series signals of the pulse interval and the blood flow; and
calculates, as a feature value, a mean, variations, or a concentration level of each of the calculated distributions.

(Supplementary note 12)

A physiological state estimation method performed by a computer, comprising:

dividing, by the computer, a time-series signal representing the physiological state of the living body into a plurality of frequency bands and calculating a feature value from a time-series variation in amplitude and a time-series variation in frequency in each of the frequency bands of the time-series signal;

calculating, by the computer, a shift vector representing the physiological state of the living body in a multidimensional vector space composed of the calculated feature values and indicating a change in the physiological state of the living body in the multidimensional vector space; and

combining, by the computer, shift vectors before and after an arbitrary physiological state change by using shift vectors before and after a desired environmental stimulus stored in a storage unit configured to store shift vectors before and after a plurality of environmental stimuli, and estimating the physiological state of the living body based on a combination ratio of the combined shift vectors.

(Supplementary note 13)

A physiological state estimation program performed by a computer, configured to cause the computer to perform:

divide a time-series signal representing the physiological state of the living body into a plurality of frequency bands and calculate a feature value from a time-series variation in amplitude and a time-series variation in frequency in each of the frequency bands of the time-series signal;

calculate a shift vector representing the physiological state of the living body in a multidimensional vector space composed of the calculated feature values and indicating a change in the physiological state of the living body in the multidimensional vector space; and

combine shift vectors before and after an arbitrary physiological state change by using shift vectors before and after a desired environmental stimulus stored in a storage unit configured to store shift vectors before and after a plurality of environmental stimuli, and estimate the physiological state of the living body based on a combination ratio of the combined shift vectors.

## Claims

1. A physiological state estimation system comprising:

a feature value calculation unit (201) configured to divide a time-series signal representing the physiological state of the living body into a plurality of frequency bands and calculate a feature value from a time-series variation in amplitude and a time-series variation in frequency in each of the frequency bands of the time-series signal;

a shift vector calculation unit (202) configured to calculate a shift vector representing the physiological state of the living body in a multidimensional vector space composed of the calculated feature values and indicating a change in the physiological state of the living body in the multidimensional vector space;

a storage unit (30) configured to store shift vectors before and after a plurality of environmental stimuli; and

a physiological state estimation unit (203) configured to combine shift vectors before and after an arbitrary physiological state change by using shift vectors before and after a desired environmental stimulus stored in the storage unit, and estimate the physiological state of the living body based on a combination ratio of the combined shift vectors.

2. The physiological state estimation system according to claim 1, wherein the shift vectors stored in the storage unit are obtained by:

calculating positional coordinates in the multidimensional vector space of feature values based on time-series signals representing physiological states before and after a plurality of types of environmental stimuli for each of a plurality of persons or each of a plurality of times; and

averaging, for each environmental stimulus, a shift vector from before the environmental stimulus to after the environmental stimulus in a state where the change in the physiological state of the living body between before and after the environmental stimulus becomes statistically significant.

3. The physiological state estimation system according to claim 1, wherein the feature value calculation unit (201) calculates a mean value and a standard deviation by using a plurality of feature values before an environmental stimulus stored in the storage unit, and use the calculated mean value and the standard deviation to standardize data before and after the environmental stimulus so that their mean value becomes 0 and their standard deviation becomes 1.

4. The physiological state estimation system according to claim 3, wherein the feature value calculation unit (201) calculates a first feature value by using a time-series signal representing a first physiological state, the first feature value being a standardized feature value before an environmental stimulus,

the feature value calculation unit (201) calculates a second feature value by using a time-series signal representing a second physiological state, the second feature value being a standardized feature value after the environmental stimulus,
the shift vector calculation unit (202) calculates a shift vector representing a change from the first physiological state to the second physiological state in the multidimensional vector space based on the first and second feature values, and
the physiological state estimation unit (203) expresses the shift vector representing the change from the first physiological state to the second physiological state by a linear combination using a shift vector stored in the storage unit, and outputs a ratio of a combination coefficient of the linear combination as an estimated value indicating a change in the physiological state of the living body.

5. The physiological state estimation system according to claim 4, wherein the feature value calculation unit (201) calculates instantaneous amplitudes and instantaneous frequencies of a signal waveform representing a physiological state in a certain frequency band, and calculates a mean value and a standard deviation obtained by adding up calculated instantaneous amplitudes and instantaneous frequencies in a section that is regarded as a physiologically steady state as the feature values.

6. The physiological state estimation system according to claim 5, wherein the frequency band represents the physiological state of the living body, and the feature value calculation unit (201) calculates the feature value for at least four frequency bands, i.e., HF (0.15 to 0.4 Hz) representing a respiratory cycle of the living body, LF (0.04 to 0.15 Hz) representing a blood pressure fluctuation cycle of the living body, and VLF 1 (15 to 40 mHz) and VLF 2 (4 to 15 mHz) representing fluctuations in an autonomic nervous system of the living body.

7. The physiological state estimation system according to claim 6, wherein the frequency band includes ULF 1 (0.15 to 0.4 mHz) and ULF 2 (0.04 to 0.15 mHz), and
the feature value calculation unit (201) calculates the feature value for at least one of the ULF 1 and the ULF 2.

8. The physiological state estimation system according to claim 6, wherein the frequency band includes frequency bands $\delta$ (0.4 to 4 Hz), $\theta$ (4 to 8 Hz), $\alpha$ (8 to 13 Hz), $\beta$ (13 to 30 Hz), and $\gamma$ (30 Hz and higher), and
the feature value calculation unit (201) calculates the feature value for at least one of the bands $\delta$, $\theta$, $\alpha$, $\beta$ and $\gamma$.

9. The physiological state estimation system according to claim 3, wherein when variance variations of the standardized feature value before and after the environmental stimulus stored in the storage unit is larger than a predetermined threshold, the shift vector calculation unit (202) converts a feature value space into an orthogonal space and calculates the shift vector.

10. The physiological state estimation system according to any one of claims 1 to 3, wherein when there are two shift vectors orthogonal to each other among shift vectors resulting from an environmental stimulus, the physiological state estimation unit (203) calculates a base vector by rotation-transforming a feature value space so that directions of arbitrary two vectors of a multidimensional eigenvalue vector matrix obtained by converting the feature value space before a plurality of environmental stimulus into an orthogonal space coincide with directions of the two shift vectors orthogonal to each other, and estimates a physiological state with inner product values of shift vectors indicating a change from a first physiological state to a second physiological state and respective base vectors, the inner product values being calculated by using a matrix of the calculated base vectors.

11. The physiological state estimation system according to claim 4, wherein the feature value calculation unit (201):

divides each of time-series signals of a pulse interval and a blood flow acquired from the living body into a plurality of frequency bands;

calculates, for each of the frequency bands, a distribution of instantaneous amplitudes and a distribution of instantaneous frequencies of the time-series signals of the pulse interval and the blood flow, and calculates a distribution of instantaneous phase differences between the time-series signals of the pulse interval and the blood flow; and

calculates, as a feature value, a mean, variations, or a concentration level of each of the calculated distributions.

12. A physiological state estimation method performed by a computer, comprising:

dividing, by the computer, a time-series signal representing the physiological state of the living body into a plurality of frequency bands and calculating a feature value from a time-series variation in amplitude and a time-series variation in frequency in each of the frequency bands of the time-series signal;

calculating, by the computer, a shift vector representing the physiological state of the living body in a multidimensional vector space composed of the calculated feature values and indicating a change in the physiological state of the living body in the multidimensional vector space; and

combining, by the computer, shift vectors before and after an arbitrary physiological state change by using shift vectors before and after a desired environmental stimulus stored in a storage unit configured to store shift vectors before and after a plurality of environmental stimuli, and estimating the physiological state of the living body based on a combination ratio of the combined shift vectors.

13. A physiological state estimation program (200) performed by a computer, configured to cause the computer to perform:

divide a time-series signal representing the physiological state of the living body into a plurality of frequency bands and calculate a feature value from a time-series variation in amplitude and a time-series variation in frequency in each of the frequency bands of the time-series signal;

calculate a shift vector representing the physiological state of the living body in a multidimensional vector space composed of the calculated feature values and indicating a change in the physiological state of the living body in the multidimensional vector space; and

combine shift vectors before and after an arbitrary physiological state change by using shift vectors before and after a desired environmental stimulus stored in a storage unit configured to store shift vectors before and after a plurality of environmental stimuli, and estimate the physiological state of the living body based on a combination ratio of the combined shift vectors.

1

PHYSIOLOGICAL STATE ESTIMATION APPARATUS

10

CALCULATION
APPARATUS

11

COMMUNICATION
INTERFACE

100

DATABASE
GENERATION
PROGRAM

200

PHYSIOLOGICAL
STATE ESTIMATION
PROGRAM

13

DISPLAY
DEVICE

12

STORAGE
DEVICE

Fig. 1

100

DATABASE GENERATION PROGRAM

101

102

30

LIVING-BODY INFORMATION
BEFORE AND AFTER
ENVIRONMENTAL STIMULUS

FEATURE VALUE
CALCULATION UNIT

SHIFT VECTOR
CALCULATION UNIT

SHIFT VECTOR
DATABASE

STORE SHIFT VECTOR
FOR EACH ENVIRONMENTAL
STIMULUS

Fig. 2

200

PHYSIOLOGICAL STATE
ESTIMATION PROGRAM

201

LIVING-BODY INFORMATION
IN ARBITRARY DIFFERENT
PHYSIOLOGICAL STATE

FEATURE VALUE
CALCULATION
UNIT

202

SHIFT VECTOR
CALCULATION UNIT

203

PHYSIOLOGICAL STATE
ESTIMATION VALUE

PHYSIOLOGICAL
STATE ESTIMATION
UNIT

30

SHIFT VECTOR
DATABASE

Fig. 3

Fig. 4

START

INPUT PLURALITY OF PIECES OF LIVING-BODY INFORMATION BEFORE AND AFTER ENVIRONMENTAL STIMULUS ~ S10

CALCULATE N-DIMENSIONAL PHYSIOLOGICAL STATE FEATURE VALUE ~ S11

IS CHANGE IN FEATURE VALUE STATISTICALLY SIGNIFICANT ? S12 — NO

YES

STANDARDIZE PHYSIOLOGICAL STATE FEATURE VALUE ~ S13

ARE VARIATIONS IN VARIANCES OF FEATURE VALUE SMALL ? S14 — NO

YES

TRANSFORM FEATURE VALUES INTO THOSE IN ORTHOGONAL COORDINATE SYSTEM ~ S17

CALCULATE SHIFT VECTOR ~ S15

CALCULATE SHIFT VECTOR ~ S18

REGISTER SHIFT VECTOR IN SHIFT VECTOR DATABASE ~ S16

REGISTER SHIFT VECTOR IN SHIFT VECTOR DATABASE ~ S19

END

F i g. 5

START

INPUT LIVING-BODY INFORMATION IN ARBITRARY DIFFERENT STATE ~ S30

CALCULATE N-DIMENSIONAL FEATURE VALUE ~ S31

STANDARDIZE N-DIMENSIONAL FEATURE VALUE ~ S32

CALCULATE SHIFT VECTOR ~ S33

EXPRESS CALCULATED SHIFT VECTOR BY LINEAR COMBINATION OF PLURALITY OF KNOWN SHIFT VECTORS ~ S34

OUTPUT LINEAR COMBINATION RATIO AS ESTIMATION VALUE OF PHYCOLOGICAL STATE ~ S35

END

Fig. 6

SHIFT VECTOR COMPONENTS RESULTING FROM MENTAL FATIGUE TASK

Fig. 7

SHIFT VECTOR COMPONENTS RESULTING FROM PHYSICAL FATIGUE TASK

Fig. 8

SHIFT VECTOR COMPONENTS RESULTING FROM PLURALITY OF FATIGUE TASKS

Fig. 9

Fig. 10

d{Im(H)}/dt＝FREQUENCY

H

Re(H)＝AMPLITUDE

Fig. 11

C3

p₁q₁ (MENTAL FATIGUE)

A

b

B

b'

a

p₂q₂ (PHYSICAL FATIGUE)

C2

C1

∠ab'b = 90°

Fig. 12

Fig. 13

ak of Fp1

Fig. 14

ωk of Fp1

In δ μ/σ = 1.06/0.69 [100:105]sec

In θ μ/σ = 7.28/0.81 [100:105]sec

In α μ/σ = 8.7/1.07 [100:105]sec

In β μ/σ = 21.62/3.85 [100:105]sec

In Y μ/σ = 60.21/8.23 [100:105]sec

Fig. 15

CIRCLE HAVING
RADIUS OF 1

$$S = |b| \times \sin(\delta)$$

$$|d|/|b| = |c|/s = 1/s$$

$$d = b/\sin(\delta)$$

$$e = -a \times \cos(\delta)/\sin(\delta)$$

$$c = d + e$$
$$= b/\sin(\delta) - a \times \cos(\delta)/\sin(\delta)$$

Fig. 16

Europäisches
Patentamt

European
Patent Office

Office européen
des brevets

# EUROPEAN SEARCH REPORT

**Application Number**

EP 25 15 0189

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/148873 A1 (HAYASHI KEIJI [JP] ET AL) 18 May 2023 (2023-05-18) * paragraphs [0012], [0015], [0049], [0055]; figures 5,9,10-13 * | 1,12,13 | INV. A61B5/024 A61B5/329 A61B5/347 A61B5/374 A61B5/00 |
| A,D | JP 2012 239480 A (DELTA TOOLING CO LTD) 10 December 2012 (2012-12-10) * paragraph [0066] * | 1,12,13 | |
| A | SORNMO LEIF ET AL: "Spectral Analysis of Heart Rate Variability in Time-Varying Conditions and in the Presence of Confounding Factors", IEEE REVIEWS IN BIOMEDICAL ENGINEERING, IEEE, USA, vol. 17, 7 November 2022 (2022-11-07), pages 322-341, XP011958257, ISSN: 1937-3333, DOI: 10.1109/RBME.2022.3220636 [retrieved on 2022-11-08] * abstract * | 1,12,13 | |

**TECHNICAL FIELDS SEARCHED (IPC)**

A61B

The present search report has been drawn up for all claims

| Place of search | Date of completion of the search | Examiner |
|---|---|---|
| Berlin | 10 June 2025 | Clevorn, Jens |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 25 15 0189

This annex lists the patent family members relating to the patent documents cited in the above-mentioned European search report.
The members are as contained in the European Patent Office EDP file on
The European Patent Office is in no way liable for these particulars which are merely given for the purpose of information.

10-06-2025

| Patent document cited in search report | Publication date | Patent family member(s) | | Publication date |
|---|---|---|---|---|
| US 2023148873 A1 | 18-05-2023 | JP | 7563363 B2 | 08-10-2024 |
| | | JP | 2023074743 A | 30-05-2023 |
| | | US | 2023148873 A1 | 18-05-2023 |
| JP 2012239480 A | 10-12-2012 | JP | 5892678 B2 | 23-03-2016 |
| | | JP | 2012239480 A | 10-12-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012239480 A

- JP 7327363 B

**Non-patent literature cited in the description**

- **H. S. TEOH**. *Formula for Vector Rotation in Arbitrary Planes in Rn*, 17 April 2005, https://www.qfbox.info/4d/genrot.pdf